# EUROPEAN PATENT SPECIFICATION

(11) **EP 4 284 789 B1**
(45) Date of publication and mention of the grant of the patent: **16.04.2025**
(21) Application number: 22704333.8
(22) Date of filing: 31.01.2022
(51) Int. Cl.: C07D 317/54

(54) **PROCESS FOR THE PREPARATION OF 3,4-METHYLENEDIOXYPROPIOPHENONE**
VERFAHREN ZUR HERSTELLUNG VON 3,4-METHYLENDIOXYPROPIOPHENON
PROCÉDÉ DE PRÉPARATION DE 3,4-MÉTHYLÈNEDIOXYPROPIOPHÉNONE

(30) Priority: 29.01.2021 WO PCT/EP2021/052187
(43) Date of publication of application: 06.12.2023
(73) Proprietor: ENDURA S.p.A., 40121 Bologna (IT)
(72) Inventor: LUCARELLI, Carlo, 40122 Bologna (IT); TABANELLI, Tommaso, 48033 Cotignola (RA) (IT); VACCARI, Angelo, 40127 Bologna (IT); EBERLE, Martina, 36013 Piovene Rocchette (VI) (IT); MARCHIORO, Carla, 37069 Villafranca di Verona (VR) (IT); CAMPANATI, Matteo, 40137 Bologna (IT); BILLI, Stefano, 48018 Faenza (RA) (IT); GUERRINI, Alberto, 48123 RAVENNA (IT); SCHIAROLI, Nicola, 60019 Senigallia (IT)
(74) Representative: Cattaneo, Elisabetta
(86) International application number: PCT/EP2022/052218
(87) International publication number: WO 2022/162223

(56) References cited:
- WO-A1-2020/174271
- PANDE MANOJ A. ET AL: "Acylation of Aromatic Ethers Using Different Carboxylic Acid Anhydrides as Acylating Agents in the Presence of Nontoxic, Noncorrosive Resin Amberlyst 15 as a Solid Acid Catalyst", SYNTHETIC COMMUNICATIONS, vol. 41, no. 5, 7 February 2011 (2011-02-07), US, pages 754 - 761, XP055826451, ISSN: 0039-7911, DOI: 10.1080/00397911003644407
- SARTORI GIOVANNI ET AL: "Use of Solid Catalysts in Friedel-Crafts Acylation Reactions +", vol. 106, no. 3, 1 March 2006 (2006-03-01), US, pages 1077 - 1104, XP055826450, ISSN: 0009-2665, Retrieved from the Internet <URL:https://pubs.acs.org/doi/pdf/10.1021/cr040695c> DOI: 10.1021/cr040695c
- GELBARD G: "Organic synthesis by catalysis with ion-exchange resins", INDUSTRIAL & ENGINEERING CHEMISTRY RESEARCH, AMERICAN CHEMICAL SOCIETY, vol. 44, no. 23, 9 November 2005 (2005-11-09), pages 8468 - 8498, XP002551927, ISSN: 0888-5885, [retrieved on 20051014], DOI: 10.1021/IE0580405
- WANG BAO-HE ET AL: "ZnCl2-modified ion exchange resin as an efficient catalyst for the bisphenol-A production", CHINESE CHEMICAL LETTERS, ELSEVIER, AMSTERDAM, NL, vol. 25, no. 11, 13 June 2014 (2014-06-13), pages 1423 - 1427, XP029092560, ISSN: 1001-8417, DOI: 10.1016/J.CCLET.2014.06.004

## Description

### FIELD OF THE INVENTION

The present invention relates to a new and inventive process for the preparation of 3,4-methylenedioxypropiophenone also known as 1-(1,3-benzodioxol-5-yl)-1-propanone as below represented.

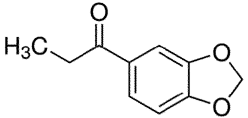

Such a process is an improved and commercially viable process working in solvent-free conditions and with a heterogeneous catalyst.

### BACKGROUND

Alkylenedioxybenzene derivatives have ganied much importance in the pharmaceutical, pesticide, biocide, performer, and food sectors due to their application as the final product or the intermediate of a desired final product.

For example, compounds having insecticidal activity containing the benzo[1,3]dioxole group have been described in numerous publications. Safrole and isosafrole are used in performary and specifically isosafrole is used for the synthesis of piperonal (benzo[1,3]dioxole-5-carboxyaldehyde) used for producing perfumes and aromas. Among aryl ketones, 3,4-Methylenedioxypropiophenone also known as 1-(1,3-benzodioxol-5-yl)-1-propanone is a crucial compound and an intermediate in the above fields.

In EP1140894B1 a method for the preparation of 5-(α-hydroxyalkyl)benzo[1,3]dioxoles is described, which provides for a step of preparation of the acyl derivative. Specifically the described process comprised a first step a) of preparing benzo[1,3]dioxole by reacting from 1,2-dihydroxybenzene and methylene chloride in a suitable solvent, followed by a step b) of reacting the so obianed benzo[1,3]dioxole with propionic anhydride in the presence of perchloric acid as acylation catalyst. In the description the proposed acylation catalysts were ZnO, ZnCl₂, FeCl₂, FeCl₃, FeSO₄, Fe₂(SO₄)₃, FeO, Fe₂O₃, H₃PO₄ and HClO₄.

Drawbacks associated to this kind of catalysts are reported in WO2020/174271. In this International application it is underlined that all these catalysts are toxic and corrosive and generally produce high amounts of hydrochloric acid in the effluent with a consequent huge amount of sludge, thus rendering the process highly polluting and environmentally harmful.

The same document thus proposes the use of an alkyl sulphonic acid to easily separate the unreacted carboxylic acid and unreacted substrate, that can be reused.

In example 14 of WO2020/174271, 3,4-methylenedioxypropiophenone is prepared by charging firstly methane sulphonic acid and propionic anhydride, and after cooling by adding 3,4-methylendioxybenzene. The reaction was maintained at 0°C to 5°C for 4 hours and after completion of the reaction, the reaction mass was diluted with water and the obtained 3,4-methylenedioxypropiophenone and the unreacted 3,4-methylenedioxybenzene were extracted with toluene. Distillation of the toluene layer allowed to recover unreacted 3,4-methylenedioxybenzene and to obtain 3,4-methylenedioxypropiophenone with a gas purity of 98%. They also recover in the aqueous phase 63 g of propionic acid

The process therein described teaches the use of an excess of the homogenous catalyst, i.e. methane sulfonic acid, that needs to be recovered. In fact, the process provides for an extraction in water/toluene in order to recover not only the wished product (in toluene) but also the catalyst. The inventors studied the process and noted that the prior art process of WO2020/174271 was able to obtain higher 3,4-methylendioxybenzene conversion and yielded into the target product in view of the use of an excess of catalyst and very stringent condition of reaction (between 0 and 5°C for four hours). Furthermore, it is well known that methyl sulphonic acid is corrosive for metals and difficult to manipulate even if used in industry.

It is also known that 3,4 methylendioxybenzene, being an aromatic cyclic ether, is characterized by a partial deactivation of the ring due to the bending of the group - CH2- in bridge position with respect to the two oxygen atoms (FT-IR Investigation of Methoxy Substituted Benzenes Adsorbed on Solid Acid Catalysts". J. Phys. Chem. C 2012, 116, 21308-21317. dx.doi.org/10.1021/jp3023056. "Anomeric Effect in 1,3-Dioxole: A Theoretical Study". J. Am. Chem. Soc. 1996, 118, 9850-9854.).Therefore stringent and drastic conditions for the acylation reaction as reported in WO2020/174271 are usually necessary.

Furthermore, as reported in WO 2018/150230, paragraph [0016], the acylation of alkylenedioxybenzene compounds using conventional processes results in synthesis of the end product in low yield and low purity, as -O-(CH2)m-O- ring of the reactant and product are very susceptible to cleavage under acid conditions.

Therefore, the object of the present invention is firstly to provide an economical, efficient, and environmentally friendly process for preparing 3,4-methylenedioxypropiophenone also known as 1-(1,3-benzodioxol-5-yl)-1-propanone, thus overcoming the drawbacks of the prior art process, and secondly acylating it with good yield and purity.

### SUMMARY OF THE INVENTION

Notwithstanding the fact that the heterogeneous catalysts, specifically those based on sulphonated resins, are known for being very less active in catalyzing the acylation of substrates, even for open heterocycles as reported for example in "*Perfluorinated nafion-modified SBA- 15 material for catalytic acylation of anisole",* Applied Catalyst A: General F.Martinez, G.Morales, A. Martin, R van Grieken, wherein many difficulties in acylating anisole are reported, the inventors found out that the heterogeneous catalysts in specific granule size are capable not only to increase the conversion from the starting material, i.e. 3,4 methylendioxybenzene, but also to increase its selectivity. The inventors in fact found out that the specific size of the granules/particles of the specific sulphonated heterogeneous catalysts surprisingly increased the selectivity that usually decreases not only with the increase of the active surfaces of the particles/granules of the heterogeneous catalysts, but also with the increase of the aromatic substrate conversion due to the promotion of the consecutive reaction over the target product.

Therefore, the Applicant has surprisingly found out that an innovative family of heterogenous catalysts having specific particle sizes that could be efficiently used for the acylation of 3,4 methylendioxybenzene. These micronized catalysts not only were capable to increase the conversion, but also the selectivity of the acylation reaction on the substrate 3,4 methylendioxybenzene, that is a closed heterocycle.

Therefore the invention relates to a process for the preparation of 1-(1,3-benzodioxol-5-yl)-1-propanone comprising the step of reacting with propionic anhydride in the presence of a bulk or supported catalyst, being said catalyst selected from the group consisting of a sulphonated cross-linked divinylbenzene resin, a sulphonated cross-linked polystyrene-divinylbenzene resin partially exchanged with iron, zinc or gallium, a perfluorinated sulphonic resin, and a perfluorinated sulphonic resin partially exchanged with iron, zinc or gallium, wherein said catalyst has an average particle size from 1 µm to 300 µm, preferably from 1 µm to 180 µm, more preferably from 1 µm to 100 µm as measured with DLS (Dynamic light scattering) or a granulometer.

When in the present invention the definition "particle size" is used is meant the average diameter of the single particle/granule of the final catalyst.

In an advantageous embodiment of the inventive process the catalyst has an average particle size from 40 µm to 100 µm.

In a preferred embodiment the catalyst is a supported catalyst on oxides, preferably supported on silica, zirconia, titania or alumina, more preferably silica.

The inventors hence surprisingly noted that the use of bulk or supported resins characterized by the presence of a sulfonic acid functional group showed peculiar catalytic activity in the acylation of 3,4 methylendioxybenzene with propionic anhydride. Furthermore, the inventors noted that the process of the acylation of 3,4-methylenedioxybenzene was extremely efficient when the catalyst was in form of powder, both used as bulk catalyst and as supported catalyst.

Advantageously, the catalyst of the process of the invention can be recovered, regenerated and recycled for starting again the reaction.

The herein used catalysts are suitable to being applied in both batch and continuous flow apparatus, leading to the selective formation of 3,4-methylenedioxypropiophenone.

In an advantageous embodiment, the process for preparing of 1-(1,3-benzodioxol-5-yl)-1-propanone is a continuous process.

From the above it is evident that the process of the invention is not only environmentally friendly with less severe conditions of operation with respect to the prior art process, but also extremely efficient for the production of 1-(1,3-benzodioxol-5-yl)-1-propanone with the possibility to work also in a continuous way.

### BRIEF DESCRIPTION OF THE DRAWINGS

Figure 1 represents the results of example 7.
Figure 2 represents the results of example 23.

### DETAILED DESCRIPTION OF THE INVENTION

The invention hence relates to a process for the preparation of 1-(1,3-benzodioxol-5-yl)-1-propanone comprising the step of reacting 3,4 methylendioxybenzene with propionic anhydride in the presence of a bulk or supported catalyst, being said catalyst selected from the group of a sulphonated cross-linked polystyrene-divinylbenzene resin, a sulphonated cross-linked polystyrene-divinylbenzene resin partially exchanged with iron, zinc or gallium, a perfluorinated sulphonic resin, and a perfluorinated sulphonic resin partially exchanged with iron, zinc or gallium, wherein said catalyst has an average particle size from 1 µm to 300 µm, preferably from 1 µm to 180 µm, more preferably from 1 µm to 100 µm as measured with DLS (Dynamic light scattering) or a granulometer.

When in the present invention the definition "particle size" is used is meant the average diameter of the single particle/granule of the final catalyst.

In an advantageous embodiment of the inventive process the catalyst has an average particle size from 40 µm to 100 µm.

The invention comprises the step of acylation reaction of 3,4 methylendioxybenzene in the presence of a bulk or supported catalyst selected from the group of a sulphonated cross-linked polystyrene-divinylbenzene resin, a sulphonated cross-linked polystyrene-divinylbenzene resin partially exchanged with iron, zinc or gallium, a perfluorinated sulphonic resin, and a perfluorinated sulphonic resin partially exchanged with iron, zinc or gallium, wherein said catalyst has an average particle size from 1 µm to 300 µm, preferably from 1 µm to 180 µm, more preferably from 1 µm to 100 µm as measured with DLS (Dynamic light scattering) or a granulometer.

All these catalysts are defined as heterogenous catalysts.

As it will be clearer from the experimental part the inventors tried to use the heterogeneous catalysts as available in the market directly in acylating 3,4 methylendioxybenzene, but the attempt leads to very poor results, specifically at low temperatures. The inventors found out that, when the catalyst has an average particle size from 1 µm to 300 µm, preferably from 1 µm to 180 µm, more preferably from 1 µm to 100 µm as measured with DLS (Dynamic light scattering) or a granulometer the acylation of MDB was possible with surprising results.

The catalyst can be a sulphonated cross-linked polystyrene-divinyl benzene resin used as bulk or supported on silica. Preferably, the sulphonated cross-linked polystyrene-divinyl benzene resin is characterized an acid loading expressed as mmol of group - SO₃H per g of material in the range from 2 to 6, more preferably 2.5 to 5.5, still more preferably about 5.

The catalyst can be a perfluorinated sulphonic acid resins used as bulk or supported on silica. Preferably the perfluorinated sulphonic acid resin is characterized by an acid loading expressed as mmol of group -SO₃H per g of material in the range from 0.2 to 2, more preferably from 0.7 to 1.6, still more preferably the catalyst has an acid loading of about 1.2.

In a preferred embodiment the catalysts above indicated are commercially available catalysts that are subjected to a micronization step in order to have particle size in the range from 1 µm to 300 µm, preferably from 1 µm to 180 µm, more preferably from 1 µm to 100 µm as measured with DLS (Dynamic light scattering) or a granulometer.

The sulphonated cross-linked polystyrene-divinyl benzene resin can be the product Amberlyst, an exchange ion resin available from Rohm and Haas Company. These resins can be the product named as:
- Amberlyst 15 having an acid loading (expressed as mmol of group -SO₃H per g of material) of 4.7 (https://www.sigmaaldrich.com/catalog/product/ aldrich/ 216399),
- Amberlyst 36 having an acid loading (expressed as mmol of group -SO₃H per g of material) of 5.4 (Sibao Liu and others, 'Renewable Lubricants with Tailored Molecular Architecture', Science Advances, 5.2 (2019) (https://doi.org/10.1126/sciadv. aav5487)),
- Amberlyst 39 having an acid loading (expressed as mmol of group -SO₃H per g of material) of 5 (Tomasz Komoń and others, 'Esterification of Acrylic Acid with 2-Ethylhexan-1-Ol: Thermodynamic and Kinetic Study', Applied Catalysis A: General, 451 (2013), 127-36 (https://doi.org/10.1016/j.apcata.2012.11.018)) or
- Amberlyst 70 having an acid loading (expressed as mmol of group -SO₃H per g of material) of 2.55 (Tomasz Komoń and others, 'Esterification of Acrylic Acid with 2-Ethylhexan-1-Ol: Thermodynamic and Kinetic Study', Applied Catalysis A: General, 451 (2013), 127-36 (https://doi.org/10.1016/j.apcata.2012.11.018)).

The perfluorinated sulphonic acid resin can be the product Aquivion, a perfluorinated resin having the following structure

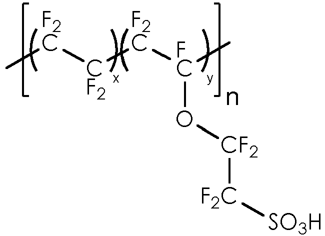

The presence of the terminal -CF₂CF₂SO₃H groups renders the perfluorinated resin strongly acidic.

The resin Aquivion can be a commercial product named as Aquivion^{®} PW79S that is a coarse, acid perfluorinated resin powder based on the short-side-chain (SSC) copolymer of Tetrafluoroethylene and the Sulfonyl Fluoride Vinyl Ether (SFVE) CF₂=CF-O-(CF₂)₂-SO₂F produced by Solvay and having an acid loading of 1.27.

The resin Aquivion can be a commercial product named as Aquivion^{®} P98-S, that is perfluorinated pellets in the sulfonyl fluoride (-SO2F) form that exhibit an Equivalent Weight (EW) of 980 g/eq. This perfluorinated resin is based on the unique Short Side Chain copolymer of Tetrafluoroethylene (TFE) and Sulfonyl Fluoride Vinyl Ether (SFVE) CF₂=CF-O-(CF₂)₂-SO₂F produced by Solvay and having an acid loading expressed as mmol of group -SO₃H per g of material of 1.02.

The resin Aquivion can be a commercial product named as Aquivion^{®} PW87-S, that is perfluorinated resin in form of powder. This perfluorinated resin is based on the unique Short Side Chain copolymer of Tetrafluoroethylene (TFE) and Sulfonyl Fluoride Vinyl Ether (SFVE) CF₂=CF-O-(CF₂)₂-SO₂F produced by Solvay and having an acid loading expressed as mmol of group -SO₃H per g of material of 1.15.

The use of other resins Aquivion available in the market or as in the present experimental part is contemplated according to the present invention.

The perfluorinated sulphonic acid resin can be the product Nafion, a perfluorinated resin having the following structure

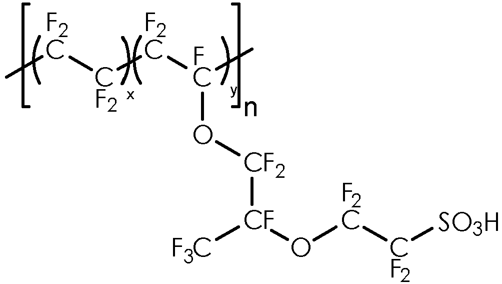

Nafion's unique ionic properties are a result of incorporating perfluorovinyl ether groups terminated with sulfonate groups onto a tetrafluoroethylene (PTFE) backbone. The resin Nafion can be a commercial product named as Nafion^{™} NR50 that is a perfluorosulfonic acid nafion resin with high thermal stability and chemical resistance in the form of pellets. This perfluorinated resin is produced by DuPont and having an acid loading expressed as mmol of group -SO₃H per g of material of 0.8.

The use of other resins Nafion available in the market or as in the present experimental part is contemplated according to the present invention.

The heterogeneous catalysts of the invention are hence characterized by the presence of sulphonic acid groups and can be also partially or completely exchanged with iron or zinc or gallium.

In fact, the catalytic material herein reported (acid resins) can be modified by exchange of the acidic proton with a metal ion selected from iron (Fe), zinc (Zn) and gallium (Ga). The procedure to be used for exchanging the hydrogen ion on a sulphonated cross-linked polystyrene-divinyl benzene resin or a perfluorinated sulphonic acid resin is reported in literature, for example in Journal of Molecular Catalysis A: Chemical 411 (2016) 257-263).

Surprisingly, the modification of the catalyst in terms of acid sites density, strength, and accessibility due to the presence of the metal ion Fe, Zn or Ga strongly affects the reactivity of the MDB in terms of conversion and selectivity towards the product.

When in the present invention the sulphoned resin catalyst is exchanged it is partially exchanged.

According to the invention a partial exchange means a percentage of hydrogen atom from 5 to 80% with a metal ion selected from Fe, Zn and Ga. Preferably the exchange percentage is from 10 to 50%, more preferably it is about 30%.

Examples of resins partially exchanged with Fe can be prepared by following the above literature and using Aquivion PW87-S in form of powders.

The final exchanged sulphonated catalysts is hence subjected to a micronization step thus having a particle size in the range from 1 µm to 300 µm, preferably from 1 µm to 180 µm, more preferably from 1 µm to 100 µm as measured with DLS (Dynamic light scattering) or a granulometer. The mentioned materials can be used as catalyst in the process of the invention supplied or supported over suitable inorganic oxides.

A suitable inorganic oxide can be silica, zirconia, titania and alumina, preferably it is silica. In particular, the supported materials may be commercially available products or be prepared as described in the literature, for example as described in the Italian patent 102018000006967.

The catalyst can be hence supported according to general procedure known in the art. A preferred procedure for preparing supported catalysts according to the invention is reported in the experimental part by way of example.

The above-mentioned resins named as Amberlyst, Aquivion and Nafion can be supported over suitable inorganic oxides. For example, Aquivion D79 can be supported on zirconia or on silica, while Aquivion PW98-S can be supported on alumina. Nafion supported on silica is also available on the market by Dupont or Merk.

The resins used as catalysts in the present invention can be supported on the inorganic oxides, preferably silica, alumina, titania, and zirconia in a percentage by weight (%) from 1 to 60, more preferably 5-30%, still more preferably from 10 to 15% by weight with respect to the total weight of the supported catalyst.

The final supported sulphonated catalysts during the synthesis steps is shaped so as to obtain a particle size ranging from 1 µm to 300 µm, preferably from 1 µm to 180 µm, more preferably from 1 µm to 100 µm as measured with DLS (Dynamic light scattering) or a granulometer.

In a preferred embodiment the catalyst used in the invention can be:
- a sulphonated cross-linked polystyrene-divinylbenzene resin partially exchanged with iron, zinc or gallium and supported on an inorganic oxide, or
- a perfluorinated sulphonic resin partially exchanged with iron, zinc or gallium and supported on an inorganic oxide.

The final supported exchanged sulphonated catalysts during the synthesis steps is shaped so as to obtain a particle size ranging 1 µm to 300 µm, preferably from 1 µm to 180 µm, more preferably from 1 µm to 100 µm as measured with DLS (Dynamic light scattering) or a granulometer.

In one embodiment of the process of the invention the step of reacting 3,4-methylendioxybenzene with propionic anhydride in the presence of a bulk or supported catalyst, previously subjected to a micronization step or produced in a precise range of particle size according to the invention (a particle size ranging from 1 µm to 300 µm, preferably from 1 µm to 180 µm, more preferably from 1 µm to 100 µm as measured with DLS (Dynamic light scattering) or a granulometer), selected from the group of a perfluorinated sulphonic resin, and a perfluorinated sulphonic resin partially exchanged with iron, zinc or gallium is carried out in the range of temperature from 50 to 200°C, more preferably 60 to 150°C, still more preferably about 80 °C. In this embodiment of the invention the reaction time is from 5 to 240min, more preferably from 5 to 120 min, still more preferably about 60 min.

In another embodiment of the process of the invention the step of reacting 3,4-methylendioxybenzene with propionic anhydride in the presence of a bulk or supported catalyst, previously subjected to a micronization step or produced in a precise range of particle size according to the invention (a particle size ranging from 1 µm to 300 µm, preferably from 1 µm to 180 µm, more preferably from 1 µm to 100 µm as measured with DLS (Dynamic light scattering) or a granulometer), selected from the group of a sulphonated cross-linked polystyrene-divinylbenzene resin, a sulphonated cross-linked polystyrene-divinylbenzene resin partially exchanged with iron, zinc or gallium is carried out in the range of temperature from 50 to 150°C, more preferably 60 to 130°C, still more preferably about 80°C or 120 °C. In this embodiment of the invention the reaction time is from 5 to 240min, more preferably from 5 to 120 min, still more preferably about 60 min.

In a preferred embodiment the process is performed in a batch reactor, more preferably under solvent free condition, loading a genuine catalytic amount of the catalyst.

The amount of catalyst is expressed as ratio between mol of H+ or of active sites per mol of limiting reagent, generally 3,4 methylendioxybenzene

In case of a bulk or supported catalyst selected from the group of a perfluorinated sulphonic resin, and a perfluorinated sulphonic resin partially exchanged with iron, zinc or gallium the ratio is preferably from 0.0001 to 1, more preferably from 0.001 to 0.04 still more preferably about 0.007.

In case of a bulk or supported catalyst selected from the group of a sulphonated cross-linked polystyrene-divinylbenzene resin, a sulphonated cross-linked polystyrene-divinylbenzene resin partially exchanged with iron, zinc or gallium the ratio is from 0.002 to 1, more preferably from 0.01 to 0.08, still more preferably about 0.03.

Advantageously the step of reacting 3,4 methylendioxybenzene with propionic anhydride is carried out preferably with a molar ratio between 3,4 methylendioxybenzene and propionic anhydride in the range from 2 to 1 to 1 to 2, more preferably in 1:1 stoichiometric ratio.

The stoichiometric ratio is more preferred, therefore with no need of a consistent excess of one of the two reagents.

In an advantageous aspect, the catalyst of the present process can be recovered, regenerated, and recycled.

In a preferred embodiment the catalyst can be recovered by filtration from the reaction medium and suspension in a diluted acid aqueous solution of nitric acid, preferably at a concentration in the range from 2%wt to 60%wt, more preferably from 10 to 40%, still more preferably of 20%wt.

In another preferred embodiment the catalyst can be recovered by filtration from the reaction medium and suspension in a diluted acid aqueous solution of hydrochloric acid, preferably at a concentration in the range from 2% wt to 30% wt, more preferably 20%wt.

The recovery of the catalyst is preferably carried out at a temperature in the range from room temperature to reflux for a time in the range from few minutes to 4 hours, more preferably 1 hour.

The recovery of the catalyst is hence a regeneration treatment. After the regeneration treatment, the catalyst can be recovered by filtration, washed with water until a neutral pH of the mother solution is achieved and dried at about 120°C for about one hour. The so obtained material, i.e. the catalyst of the invention, can be recycled for another reaction.

Furthermore, the herein reported catalytic materials are suitable to being applied in both batch and continuous flow apparatus, leading to the selective formation of 3,4-methylenedioxypropiophenone.

The inventors hence surprisingly noted that the use of bulk or supported resins having granules/particles in specific size range and characterized by the presence of sulphonic acid functional groups show peculiar catalytic activity in the acylation of 3,4 methylendioxybenzene with propionic anhydride. Furthermore, the inventors noted that the process of the acylation of 3,4 methylenedioxybenzene was extremely efficient even on a very little reactive starting material as 3,4 methylenedioxybenzene when the catalyst was in form of a powder, both used as bulk catalyst and as supported catalyst, having a particle size range from 1 µm to 300 µm, preferably from 1 µm to 180 µm, more preferably from 1 µm to 100 µm as measured with DLS (Dynamic light scattering) or a granulometer.

Advantageously, the catalyst of the process of the invention can be recovered, regenerated and recycled for starting again the reaction.

In an advantageous embodiment, the process for preparing of 1-(1,3-benzodioxol-5-yl)-1-propanone is a continuous process.

From the above it is evident that the process of the invention is not only environmentally friendly with less severe conditions of operation with respect to the prior art process, solvent free conditions, but also extremely efficient for the production of 1-(1,3-benzodioxol-5-yl)-1-propanone with the possibility to work also in a continuous way. Surprisingly, the process of the invention has no need of a consistent excess of one of the two reagents and showed short reaction time and relatively low reaction temperature. The process is further characterized by an easy recovery, regeneration and recycle of the catalyst.

Without being bound to any theory the inventors deem that it is of fundamental importance to cover the possibility to support the present heterogeneous catalyst over suitable supports, having with high pore volume and/or high surface area.

Features and advantages of the invention will also appear more clearly from the following non-limiting examples.

### EXPERIMENTAL PART

The following acronyms were used for indicating ingredients/products/effect in the experimental part:
MBD = 3,4-methylenedioxybenzene
AP = propionic anhydride
MDP1P = 3,4-methylenedioxypropriophenone
X= conversion
Y=yield
S=selectivity

### Example 1 Acylation of MDB with Aquivion PW87 as available on the market

2.126 grams of 3,4-methylenedioxybenzene (MBD) and 2.2924 grams of propionic anhydride were loaded inside a 25 mL two-necked round bottom flask and stirred to get a homogeneous solution. After that 0.0988 grams of catalyst (Aquivion PW87-S, corresponding to a mol ratio between active acid sites and MDB equal to 0.007), as available on the market having a particle size ranging from 0.6 mm to 1.1mm as measured with DLS (Dynamic light scattering) or a granulometer, was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID). The results were the following: MDB conversion of 40%, propionic anhydride conversion of 64%, 3,4-methylenedioxypropiophenone yield of 26% and selectivity of 64%.

### Example 2

### Preparation of 3,4-methylenedioxypropiophenone with catalyst Aquivion PW87-S at 80°C, 1 h according to the invention

2.126 grams of 3,4-methylenedioxybenzene (MBD) and 2.2924 grams of propionic anhydride were loaded inside a 25 mL two-necked round bottom flask and stirred to get a homogeneous solution. After that 0.0988 grams of catalyst (Aquivion PW87-S, corresponding to a mol ratio between active acid sites and MDB equal to 0.007), previously subjected to a micronization process to a particle size ranging from 40 µm to 100 µm as measured with DLS (Dynamic light scattering) or a granulometer, was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID). The results were the following: MDB conversion of 50%, propionic anhydride conversion of 77%, 3,4-methylenedioxypropiophenone yield of 41% and selectivity of 82%.

The inventors hence noted that the catalyst when in the specific inventive granule/particle size allowed not only a higher conversion of the initial products but also a higher selectivity, that was not expected by reducing the particle size of the catalyst.

### Example 3

### Influence of the particle size on the catalytic activity of Aquivion PW87

The influence of the catalyst particle size on the catalytic activity on the acylation of MDB was investigated as described in example 2 by changing only the particle size of the catalyst. The results are reported in the following table X.

| µm | X MDB (%) | X AP (%) | S MDP1P (%) | Y ACP (%) | Y MDP1P (%) | Y By-products |
|---|---|---|---|---|---|---|
| 841-595 | 40 | 64 | 64 | 100 | 26 | 14 |
| 595-400 | 40 | 65 | 70 | 100 | 28 | 12 |
| 400-250 | 45 | 73 | 72 | 100 | 32 | 13 |
| 250-177 | 48 | 75 | 73 | 100 | 35 | 13 |
| 177-100 | 50 | 81 | 75 | 100 | 37 | 13 |
| 100-40 | 50 | 77 | 82 | 100 | 41 | 9 |
| 40-1 | 51 | 78 | 82 | 100 | 42 | 9 |

The catalyst particle size plays a fundamental role in the reaction. The decrease of the particle size led to an improvement of the MDB conversion that mainly depends on the catalyst active surface (which increases as the particle diameters become smaller). Surprisingly, also the reaction selectivity towards MDP1P was highly improved when the catalyst particle size was decreased.

### Example 4

### Evaluation of the temperature effect in the presence of catalyst Aquivion PW87-S

Other four reactions were performed as described in example 2 by changing only the reaction temperature namely: 60°C, 80°C, 100°C and 120°C. The results are reported in Table 1.

**Table 1**

| **T (°C)** | **X MDB (%)** | **X AP (%)** | **Y MDP1P (%)** | **Y ACP (%)** | **S MDP1P (%)** | **By-products (%)** |
|---|---|---|---|---|---|---|
| *120* | *74* | *100* | *51* | *100* | *69* | *22* |
| *100* | *62* | *92* | *48* | *100* | *77* | *14* |
| *80* | *49* | *80* | *40* | *99* | *82* | *8* |
| *60* | *37* | *72* | *31* | *99* | *84* | *5* |

As in example 2, reaction conditions were the following: mol ratio between active acid sites and MDB equal to 0.007; MDB:AP=1:1; reaction time 1h.

It was noted that the increase of the reaction temperature led to an increase in the conversion of both reagents (as expected from the foster of the kinetics) however the increase of the MDP1P yield is not linear therefore a slight decline in the selectivity was observed, favoring the formation of by-products (polyalkylated and others).

At any rate, all the temperatures resulted to be efficient in having high yield balanced with the achieved selectivity.

### Example 5

### Scale-up tests to 40 ml

20.21 grams of MDB and 21.74 grams of propionic anhydride were loaded inside a 250 mL three-necked round bottom flask and stirred to get a homogeneous solution. The reagent mix was heated in an oil bath at 80°C and after that 0.9363 grams of catalyst (Aquivion PW87-S, corresponding to a mol ratio between active acid sites and MDB equal to 0.007) previously subjected to a micronization process to a particle size ranging from 40 µm to 100 µm as measured with DLS (Dynamic light scattering) or a granulometer, was inserted inside the flask which was equipped with two condensers and a thermometer. The reaction was performed under magnetic stirring (720 rpm) for 1 hour. After the reaction, the mixture was rapidly cooled in an ice bath and the reaction mixture is recovered in 500 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column, and a flame ionization detector (FID). The results were the following: MDB conversion of 53%, propionic anhydride conversion of 82%, 3,4-methylenedioxypropiophenone yield of 43% and selectivity of 81%.

It was shown that the increase of a x10 factor (in comparison with example 1) did not affect the conversion nor the yield.

### Example 6

### Scale-up to 400 mL

202.16 grams of MDB and 217.22 grams of propionic anhydride were loaded inside a 1L jacketed reactor equipped with a mechanical stirrer, reflux condenser and a thermometer. The reagent mix was heated until 80°C and after that 9.3586 grams of catalyst (Aquivion PW87-S, corresponding to a mol ratio between active acid sites and MDB equal to 0.007) previously subjected to a micronization process to a particle size ranging from 40 µm to 100 µm as measured with DLS (Dynamic light scattering) or a granulometer, was inserted inside. The reaction was performed under mechanical stirring (250 rpm) for 1 hour. After the reaction, 2mL of the reaction mixture was sampled and separated from the catalyst by filtration. 50 µL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column, and a flame ionization detector (FID). The results were the following: MDB conversion of 46%, propionic anhydride conversion of 71%, 3,4-methylenedioxypropiophenone yield of 38% and selectivity of 82%.

### Example 7

### Evaluation of the reaction time effect for PW87-S in a reaction as described in Example 6.

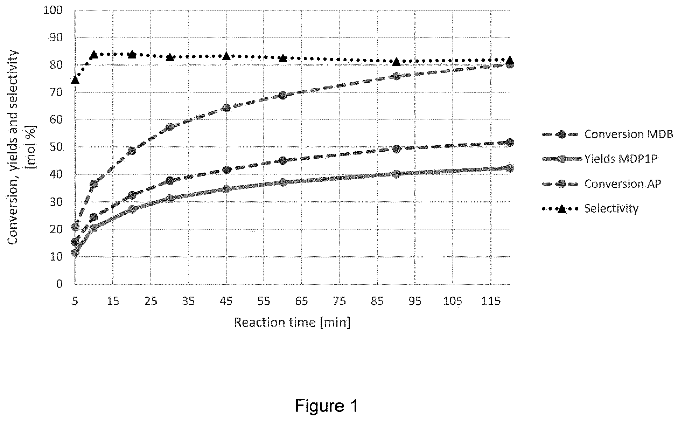

The reaction was repeated as described in example 5 with the aim of investigate the effect of reaction time on a bigger scale reactor. To do so the reaction mixture was sampled at different time as reported in example 5. The results are reported in Table 2 below and represented in Figure 1.

**Table 2**

| **Time (min)** | **X MDB (%)** | **X AP (%)** | **Y MDP1P (%)** | **Y ACP (%)** | **S MDP1P (%)** | **Y By-products (%)** |
|---|---|---|---|---|---|---|
| *5* | *15* | *21* | *11* | *23* | *75* | *4* |
| *10* | *25* | *37* | *21* | *42* | *84* | *5* |
| *20* | *32* | *49* | *27* | *53* | *84* | *6* |
| *30* | *38* | *57* | *31* | *65* | *83* | *7* |
| *45* | *42* | *64* | *35* | *74* | *83* | *8* |
| *60* | *45* | *69* | *37* | *73* | *83* | *8* |
| *90* | *49* | *76* | *40* | *85* | *81* | *9* |
| *120* | *52* | *80* | *42* | *90* | *82* | *11* |

By sampling the reaction mixture at different time it was possible to verify that during the reaction the selectivity to the desired product did not change. The conversion increased with reaction time as the yield in the desire product. The best compromise between yield and reaction time was 1h but, it was possible to obtain about the same results stopping the reaction after 30 min. In the latter case the conversion of MDB and yield in the product were few point% lower but it was possible to double the number of catalytic cycles per day increasing the overall yield. Increasing the reaction time till 2 h it was possible to increases conversion and yield without a decrease of selectivity in order to reduce the amount of reactant that had to be recycled.

### Example 8

### Evaluation of preferred conditions when Aquivion PW79 is the catalyst

2.1305 grams of MDB and 2.2879 grams of propionic anhydride were loaded inside a 25 mL two-necked round bottom flask and stirred to get a homogeneous solution. After that 0.0900 grams of catalyst (Aquivion PW79, corresponding to a molar ratio between active acid sites and MDB equal to 0.007) previously subjected to a micronization process to a particle size ranging from 40 µm to 100 µm as measured with DLS (Dynamic light scattering) or a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column, and a flame ionization detector (FID). The results were the following: MDB conversion of 64%, propionic anhydride conversion of 92%, 3,4-methylenedioxypropiophenone yield of 47% and selectivity of 73%.

### Example 9

### Acylation of MDB with catalyst Aquivion PW98 as available on the market

2.1307 grams of MDB and 2.2879 grams of propionic anhydride were loaded inside a 25 mL two-necked round bottom flask and stirred to get a homogeneous solution. After that 0.1117 grams of catalyst (Aquivion PW98, corresponding to a mol ratio between active acid sites and MDB equal to 0.007) as available on the market having a particle size ranging from 1.4 mm to 2.4 mm as measured with a granulometer, was inserted inside the liquid mixture and the flask was equipped with two condensers. The reaction mixture was then inserted in a pre-heated oil bath at 100°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column, and a flame ionization detector (FID). The results were the following: MDB conversion of 11%, propionic anhydride conversion of 18%, 3,4-methylenedioxypropiophenone yield of 7% and selectivity of 55%.

### Example 10

### Acylation of MDB with catalyst Aquivion PW98 according to the invention

2.1307 grams of MDB and 2.2879 grams of propionic anhydride were loaded inside a 25 mL two-necked round bottom flask and stirred to get a homogeneous solution. After that 0.1117 grams of catalyst (Aquivion PW98, corresponding to a mol ratio between active acid sites and MDB equal to 0.007) previously subjected to a micronization process to a particle size ranging from 40 µm to 100 µm as measured with DLS (Dynamic light scattering) or a granulometer, was inserted inside the liquid mixture and the flask was equipped with two condensers. The reaction mixture was then inserted in a pre-heated oil bath at 100°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column, and a flame ionization detector (FID). The results were the following: MDB conversion of 40%, propionic anhydride conversion of 64%, 3,4-methylenedioxypropiophenone yield of 29% and selectivity of 72%.

### Example 11

### Process of the invention with catalyst Aquivion D79 supported over silica

2.1282 grams of MDB and 2.2860 grams of propionic anhydride were loaded inside a 25 mL two-necked round bottom flask and stirred to get a homogeneous solution. After that 0.2999 grams of catalyst (Aquivion D79 30% over silica, corresponding to a mol ratio between active acid sites and MDB equal to 0.007) characterized by a particle size ranging from 40 µm to 100 µm as measured with DLS (Dynamic light scattering) or a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column, and a flame ionization detector (FID). The results were the following: MDB conversion of 65%, propionic anhydride conversion of 95%, 3,4-methylenedioxypropiophenone yield of 47% and selectivity of 72%.

### Example 12

### Lab scale regeneration general procedure of the catalyst in the invention process

Firstly, the catalyst was separated from the reaction solution by filtrating on a Büchner funnel fitted with a filtering aid. Considering 0.1g of exhaust catalyst, this was washed with acetone HPLC (25 mL) in order to remove all the weakly adsorbed reactants and products remained after reaction and finely pulverized in a mortar. After that the catalyst was inserted inside the flask equipped with two condensers. 5mL of 20wt% of HNO₃ (or HCl) solution were then used to regenerate the catalyst under reflux for 1h. The resultant suspension was then cooled down and separated from the solution by filtration on a Büchner funnel fitted with a filtering aid. The catalyst was washed with water until neutral pH and afterwards washed again with acetone HPLC. Before the catalytic test, the catalyst was dried into an oven at 120 °C for 1h.

### Example 13

### Recycle test with catalyst PW87-S, regeneration with nitric acid

The catalyst from Example 5 was regenerated as in Example 12 with 50mL of solution of HNO₃ 20%w/w at 100°C for 1h.

13.61 grams of MDB and 14.57 grams of propionic anhydride were loaded inside a 250 mL three-necked round bottom flask and stirred to get a homogeneous solution. The reagent mix was heated in an oil bath at 80°C and after that 0.6299 grams of regenerated catalyst (Aquivion PW87-S, corresponding to a mol ratio between active acid sites and MDB equal to 0.007), previously subjected to a micronization process to a particle size ranging from 40 µm to 100 µm as measured with DLS (Dynamic light scattering) or a granulometer, was inserted inside the flask which was equipped with two condensers and a thermometer. The reaction was performed under magnetic stirring (720 rpm) for 1 hour. After the reaction, 2mL of the reaction mixture was sampled and separated from the catalyst by filtration. 50 µL of the mixture is dilute in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID).

Catalyst recovery and regeneration as described in Example 12 were done for five times in order to test the recyclability of the material in few catalytic runs. The results are reported in the following table 3.

**Table 3**

| Cycle | X MDB (%) | X AP (%) | Y MDP1P (%) | Y ACP (%) | S MDP1P (%) | Y By-products (%) |
|---|---|---|---|---|---|---|
| | 53 | 82 | 43 | 100 | 81 | 9 |
| 1 | 50 | 73 | 38 | 77 | 76 | 11 |
| 2 | 49 | 75 | 39 | 81 | 79 | 10 |
| 3 | 46 | 71 | 37 | 80 | 80 | 9 |
| 4 | 51 | 75 | 40 | 76 | 78 | 10 |
| 5 | 50 | 79 | 38 | 99 | 76 | 11 |

| | | | | | | |
|---|---|---|---|---|---|---|
| Reaction conditions: mol ratio between active acid sites and MDB equal to 0.007; MDB:AP=1:1; reaction temperature 80°C; reaction time 1h. | | | | | | |

The catalyst was recovered and recycled several times by using nitric acid with evident advantages in terms of process efficiency.

### Example 14

### Recycle test with catalyst Aquivion PW87-S, regeneration with hydrochloric acid

2.1284 grams of MDB and 2.2935 grams of propionic anhydride were loaded inside a 25 mL two-necked round bottom flask and stirred to get a homogeneous solution. After that 0.0990 grams of catalyst (Aquivion PW87-S, corresponding to a mol ratio between active acid sites and MDB equal to 0.007) previously subjected to a micronization process to a particle size ranging from 40 µm to 100 µm as measured with DLS (Dynamic light scattering) or a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID). After that, the catalyst was regenerated as in Example 12 with 5mL of solution of HCl 20%w/w at 100°C for 1h, recovered, washed and dried as previously reported and tested in the same conditions for the acylation reaction. The results were reported in the following table 4.

**Table 4**

| **Cycle** | **X MDB (%)** | **X AP (%)** | **Y MDP1P (%)** | **Y ACP (%)** | **S MDP1P (%)** | **Y By-products (%)** |
|---|---|---|---|---|---|---|
| | *73* | *100* | *44* | *100* | *60* | *28* |
| *1* | *68* | *98* | *42* | *100* | *62* | *26* |

| | | | | | | |
|---|---|---|---|---|---|---|
| Reaction conditions: molar ratio between active acid sites and MDB equal to 0.007; MDB:AP=1:1; reaction temperature 120°C; reaction time 1h. | | | | | | |

The catalyst was recovered and recycled by using hydrochloric acid with evident advantages in terms of process efficiency.

### Example 15

### Effect of the molar ratios between the reagents over the catalyst Aquivion PW87-S as micronized according to the invention

Other four reactions were performed as described in example 2 by changing only the molar ratio between reagents, mol MDB/mol AP ranging from 1:1, 1.4:1, 2:1 and 1:2. The results were reported in the following Table 5.

**Table 5**

| **Catalyst (gr)** | **MDB (gr)** | **AP (gr)** | **MDB: AP** | **X MDB (%)** | **X AP (%)** | **Y MDP1 P (%)** | **Y AC P (%)** | **S MDP1 P (%)** | **Y By-products (%)** |
|---|---|---|---|---|---|---|---|---|---|
| 0.0989 | 2.1270 | 4.5595 | 1:2 | 60 | 69 | 39 | 87 | 65 | 20 |
| 0.0990 | 2.1311 | 2.2873 | 1:1 | 49 | 80 | 40 | 99 | 81 | 10 |
| 0.0986 | 2.9792 | 2.2992 | 1.4:1 | 36 | 69 | 30 | 73 | 83 | 6 |
| 0.0990 | 4.2561 | 2.2801 | 2:1 | 16 | 45 | 13 | 49 | 81 | 3 |

| | | | | | | | | | |
|---|---|---|---|---|---|---|---|---|---|
| Reaction conditions: molar ratio between active acid sites and limiting reagent equal to 0.007; temperature 80°C; reaction time 1h. | | | | | | | | | |

The increase of the excess of AP led to a higher conversion value for 3,4 methylendioxybenzene but with a detrimental effect on the selectivity, on the other hand working with a slight excess of MDB led to a more selective conversion of AP toward the target product. In any case, the best compromise between MDB conversion and selectivity toward the MDP1P product was the stoichiometric amount of the reagents.

### Example 16

### Effect of the catalyst loading with Aquivion PW87-S as function of molar ratio between active acid sites and limiting reagent.

Other three reactions were performed as described in example 2 by changing only the molar ratio between active acid sites and limiting reagent namely: 0.003, 0.007 and 0.014. The catalyst was subjected to a micronization process to a particle size ranging from 40 µm to 100 µm as measured with DLS (Dynamic light scattering) or a granulometer. The results are in the following Table 6.

**Table 6**

| **catalyst (gr)** | **MDB (gr)** | **AP (gr)** | **Mol H+/mol reag limit** | **X MDB (%)** | **X AP (%)** | **Y MDP1 P (%)** | **Y ACP (%)** | **S MDP1 P (%)** | **Y By-product s (%)** |
|---|---|---|---|---|---|---|---|---|---|
| 0.0494 | 2.1293 | 2.2866 | 0.003 | 18 | 37 | 15 | 54 | 83 | 3 |
| 0.0990 | 2.1311 | 2.2873 | 0.007 | 49 | 80 | 40 | 99 | 81 | 9 |
| 0.2121 | 2.1310 | 2.2772 | 0.014 | 57 | 95 | 46 | 100 | 81 | 16 |

| | | | | | | | | | |
|---|---|---|---|---|---|---|---|---|---|
| Reaction conditions: MDB:AP=1:1; temperature 80°C; reaction time 1h | | | | | | | | | |

The increase of the catalyst loading favoured the conversion of our reagents, with only a slight decrease in the product selectivity.

### Example 17

### Zn exchanged Aquivion D79 supported over silica.

A reaction was performed as described in example 9 by exchanging supported Aquivion D79, characterized by a particle size ranging from 40 µm to 100 µm as measured with DLS (Dynamic light scattering) or a granulometer, with Zn, specifically 100% exchanged with Zn²⁺.

The results are in the following Table 7.

**Table 7**

| **Cataly st (gr)** | **MDB (gr)** | **AP (gr)** | **Exchange** | **X MDB (%)** | **X AP (%)** | **Y MDP1P (%)** | **Y ACP (%)** | **S MDP1P (%)** | **Y By-product s (%)** |
|---|---|---|---|---|---|---|---|---|---|
| 0.3002 | 2.1308 | 2.2910 | 100%Zn²⁺ | 5 | 9 | 4 | 17 | 80 | 1 |

| | | | | | | | | | |
|---|---|---|---|---|---|---|---|---|---|
| Conditions: MDB:AP=1:1; *temperature 120°C; reaction time 1h.* | | | | | | | | | |

It was evident that the exchange with a percentage of 100% of Zn had an unfavourable effect on the reaction.

Therefore the inventors found out that only with a catalyst having a partial exchange was possible acylating MDB as shown in the below example 18. The partial exchange of the catalyst according to the invention was a percentage of hydrogen atom from 5 to 80% exchanged with a metal ion selected from Fe, Zn and Ga, preferably the exchange percentage was from 10 to 50%, more preferably it was about 30%.

### Example 18

### Fe exchanged Aquivion PW87-S

Other reactions were performed as described in example 2 at 80°C or 120°C by the exchanging Aquivion PW87-S with Fe: 50% exchanged with Fe³⁺ and 100% exchanged with Fe³⁺. The final catalyst was characterized by a particle size ranging from 40 µm to 100 µm as measured with DLS (Dynamic light scattering) or a granulometer. After a first cycle of reaction the catalyst was recovered, washed with 2-propanone and re-cycled.

The results are reported in the following Table 8.

**Table 8**

| **CY CL E** | **Temp eratu re (°C)** | **Cataly st (gr)** | **MDB (gr)** | **AP (gr)** | **Exchang e** | **X MDB (%)** | **X AP (%)** | **Y MDP1P (%)** | **Y ACP (%)** | **S MDP 1P (%)** | **Y By-products (%)** |
|---|---|---|---|---|---|---|---|---|---|---|---|
| I | 80 | 0.1007 | 2.1296 | 2.2809 | 50%Fe³⁺ | 7 | 24 | 7 | 36 | 96 | <1 |
| II | 80 | 0.0621 | 1.3174 | 1.4058 | 50%Fe³⁺ | <1 | 9 | <1 | 20 | 0 | <1 |
| I | 120 | 0.1010 | 2.1287 | 2.2795 | 100%Fe³⁺ | 5 | 11 | 1 | 11 | 16 | 3 |
| II | 120 | 0.0617 | 1.3054 | 1.4037 | 100%Fe³⁺ | <1 | <1 | <1 | 5 | 0 | <1 |

| | | | | | | | | | | | |
|---|---|---|---|---|---|---|---|---|---|---|---|
| Conditions: MDB:AP=1:1; *reaction time 1h.* | | | | | | | | | | | |

When the Aquivion is partially exchanged with an ion as Fe or Zn the conversion of MDB strongly decreases but the selectivity increases

### Example 19

### Effect of the catalyst shape

Other reactions were performed as described in example 9 at 120°C changing only the catalyst form: powder (Aquivion PW98) and pellets (8-14 mesh, Aquivion P98), the sole former was subjected to a micronization process to a particle size ranging from 40 µm to 100 µm as measured with DLS (Dynamic light scattering) or a granulometer. The results are reported in the following Table 9.

**Table 9**

| **catalyst (gr)** | **MDB (gr)** | **AP (gr)** | **Form** | **X MDB (%)** | **X AP (%)** | **Y MDP1P (%)** | **Y ACP (%)** | **S MDP1P (%)** | **Y By-products (%)** |
|---|---|---|---|---|---|---|---|---|---|
| 0.1114 | 2.1273 | 2.2855 | Powder | 63 | 94 | 42 | 100 | 67 | 22 |
| 0.1154 | 2.1282 | 2.2883 | Pellets | 18 | 26 | 10 | 31 | 56 | 8 |

| | | | | | | | | | |
|---|---|---|---|---|---|---|---|---|---|
| Reaction conditions: molar ratio between active acid sites and limiting reagent equal to 0.007; MDB:AP=1:1; temperature 120°C; reaction time 1h. | | | | | | | | | |

It was surprisingly noted that the catalyst having a particle size according to the invention showed better results in terms of yield, conversion and selectivity with respect to the same catalyst when used in pellets of 8-14 mesh.

### Example 20

### Recycle test with Aquivion PW79, regeneration with nitric acid

2.1303 grams of MDB and 2.2947 grams of propionic anhydride were loaded inside a 25 mL two-necked round bottom flask and stirred to get a homogeneous solution. After that 0.0903 grams of catalyst (Aquivion PW79, corresponding to a mol ratio between active acid sites and MDB equal to 0.007), previously subjected to a micronization process to a particle size ranging from 40 µm to 100 µm as measured with DLS (Dynamic light scattering) or a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID).

After the first run, the catalyst was regenerated as in Example 12 with 5mL of solution of HNO₃ 20%w/w at 100°C for 1h, recovered, washed, dried and finally tested in the same conditions as reported above. The results were reported in the following table 10.

**Table 10**

| **Cycle** | **X MDB (%)** | **X AP (%)** | **Y MDP1P (%)** | **Y ACP (%)** | **S MDP1P (%)** | **Y By-products (%)** |
|---|---|---|---|---|---|---|
| | *65* | *96* | *45* | *100* | *70* | *19* |
| *1* | *63* | *100* | *42* | *100* | *66* | *20* |

| | | | | | | |
|---|---|---|---|---|---|---|
| Reaction conditions: molar ratio between active acid sites and MDB equal to 0.007; MDB:AP=1:1; reaction temperature 120°C; reaction time 1h. | | | | | | |

The catalyst was recovered and recycled several times by using nitric acid with evident advantages in terms of process efficiency.

### Example 21

### Recycle test with Aquivion PW79, regeneration with hydrochloric acid

2.1288 grams of MDB and 2.2972 grams of propionic anhydride were loaded inside a 25 mL two-necked round bottom flask and stirred to get a homogeneous solution. After that 0.0902 grams of catalyst (Aquivion PW79, corresponding to a mol ratio between active acid sites and MDB equal to 0.007) previously subjected to a micronization process to a particle size ranging from 40 µm to 100 µm as measured with DLS (Dynamic light scattering) or a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID).

After the first run, the catalyst was regenerated as in Example 12 with 5mL of solution of HCl20%w/w at 100°C for 1h, recovered, washed, dried and finally tested in the same conditions as reported above. The results are reported in the following table 11.

**Table 11**

| **Cycle** | **X MDB (%)** | **X AP (%)** | **Y MDP1P (%)** | **Y ACP (%)** | **S MDP1P (%)** | **Y By-products (%)** |
|---|---|---|---|---|---|---|
| | *65* | *97* | *45* | *100* | *70* | *19* |
| *1* | *63* | *95* | *43* | *100* | *68* | *20* |

| | | | | | | |
|---|---|---|---|---|---|---|
| Reaction conditions: molar ratio between active acid sites and MDB equal to 0.007; MDB:AP=1:1; reaction temperature 120°C; reaction time 1h. | | | | | | |

The catalyst was recovered and recycled by using nitric acid with evident advantages in terms of process efficiency.

### Example 22

### Acylation of MDB with Amberlyst 39 catalyst as available on the market having particle size of 200 - 300 µm

2.1284 grams of MDB and 2.2822 grams of propionic anhydride were loaded inside a 25 mL two-necked round bottom flask and stirred to get a homogeneous solution. After that 0.1003 grams of catalyst (Amberlyst 39, corresponding to a mol ratio between active acid sites and MDB equal to 0.029) as available on the market having a particle size ranging from 200 µm to 300 µm as measured with DLS (Dynamic light scattering) or a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID). The results were the following: MDB conversion of 42%, propionic anhydride conversion of 65%, 3,4-methylenedioxypropiophenone yield of 29% and selectivity of 69%

### Example 23

### Acylation of MDB with micronized Amberlyst 30 catalyst and recycle test, regeneration with nitric acid

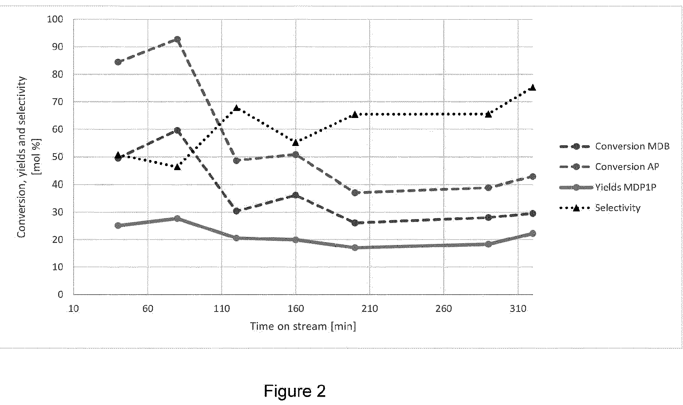

2.1284 grams of MDB and 2.2822 grams of propionic anhydride were loaded inside a 25 mL two-necked round bottom flask and stirred to get a homogeneous solution. After that 0.1003 grams of catalyst (Amberlyst 39, corresponding to a mol ratio between active acid sites and MDB equal to 0.029) previously subjected to a micronization process to a particle size ranging from 40 µm to 100 µm as measured with DLS (Dynamic light scattering) or a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID). After the first run, the catalyst was regenerated as in Example 12 with 5mL of solution of HNO₃20%w/w at 100°C for 1h, recovered, washed, dried and finally tested in the same conditions as reported above. The results are reported in the following table 12 and represented in figure 2.

**Table 12**

| **Cycle** | **X MDB (%)** | **X AP (%)** | **Y MDP1P (%)** | **Y ACP (%)** | **S MDP1P (%)** | **Y By-products (%)** |
|---|---|---|---|---|---|---|
| | 54 | 87 | 40 | 100 | 74 | 14 |
| 1 | 60 | 92 | 45 | 100 | 75 | 15 |

| | | | | | | |
|---|---|---|---|---|---|---|
| Reaction conditions: molar ratio between active acid sites and MDB equal to 0.029; MDB:AP=1:1; reaction temperature 120°C; reaction time 1h. | | | | | | |

The catalyst was recovered and recycled several times by using hydrochloric acid with evident advantages in terms of process efficiency.

The use of the catalyst in the preferred range of size particle allowed to obtain higher conversion and better selectivity.

### Example 24

### Acylation of MDB with Nafion NR50 catalyst as available on the market having particle size of 400 - 600 µm

2.1274 grams of MDB and 2.2815 grams of propionic anhydride were loaded inside a 25 mL two-necked round bottom flask and stirred to get a homogeneous solution. After that 0.1419 grams of catalyst (Nafion NR50, corresponding to a mol ratio between active acid sites and MDB equal to 0.007), as available on the market having a particle size ranging from 400 µm to 600 µm as measured with a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID). The results were the following: MDB conversion of 20%, propionic anhydride conversion of 29%, 3,4-methylenedioxypropiophenone yield of 11% and selectivity of 56%

### Example 25

### Acylation of MDB with Nafion NR50 catalyst having particle size according to the invention

As a general procedure, 2.1274 grams of MDB and 2.2815 grams of propionic anhydride were loaded inside a 25 mL two-necked round bottom flask and stirred to get a homogeneous solution. After that 0.1419 grams of catalyst (Nafion NR50, corresponding to a mol ratio between active acid sites and MDB equal to 0.007), previously subjected to a micronization process to a particle size ranging from 150 µm to 300 µm as measured with a granulometer, was inserted inside the liquid mixture and the flask was equipped with two condensers. The reaction mixture was then inserted in a pre-heated oil bath at 120°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID). After the first run, the catalyst was regenerated as in Example 12 with 5mL of solution of HNO₃20%w/w at 100°C for 1h, recovered, washed, dried and finally tested in the same conditions as reported above. The results are reported in the following table 13.

**Table 13**

| **Cycle** | **X MDB (%)** | **X AP (%)** | **Y MDP1P (%)** | **Y ACP (%)** | **S MDP1P (%)** | **Y By-products (%)** |
|---|---|---|---|---|---|---|
| | 62 | 92 | 39 | 99 | 63 | 21 |
| 1 | 62 | 93 | 40 | 97 | 64 | 21 |

| | | | | | | |
|---|---|---|---|---|---|---|
| Reaction conditions: molar ratio between active acid sites and MDB equal to 0.007; MDB:AP=1:1; reaction temperature 120°C; reaction time 1h. | | | | | | |

The inventors noted that the catalyst according to the invention allowed better conversions, yield and selectivity with respect to the same catalyst not micronized in the inventive range.

### Example 26

### Catalytic test under continuous flow conditions

The catalytic test was performed in a fixed-bed lab-scale reactor, operating under continuous-flow at atmospheric pressure. Firstly 0.1799 grams (0,4 ml) of catalyst (Aquivion PW79), previously subjected to a micronization process to a particle size ranging from 40 µm to 100 µm as measured with DLS (Dynamic light scattering) or a granulometer, mixed with carborundum (total volume 0.6 mL) was inserted inside the reactor consisting of a stainless-steel tube ¼" (with quartz wool at the beginning and the end of the reactor). The reactor was wrapped with a heating band and finally covered with insulating ceramic fabric bandages. When the system reached the temperature of 150°C the reagent mixture (composed by 26.007 grams of MDB and 28.631 grams of propionic anhydride) (1/1 mol/mol) was fed through a high precision HPLC pump at 0.05 mL/min. The reaction time started from the exit of the first drop of mixture from the outlet line of the system. Samples were taken every 10 minutes. From each sampling 50 µL of the mixture was diluted in 10 mL of acetone HPLC grade, 20 µL of octane was used as internal standard for the gas-chromatograph quantifications. The analysis of the products at the end of the reaction was carried out by a GC equipped with an HP-5 (Agilent) capillary column (the temperature ramp used consisted of 6 minutes at 35°C followed by heating with a speed of 10.00 °C/min up to 280°C), and a flame ionization detector (FID). The results corresponding to relevant time points are reported in the following Table 14.

**Table 14**

| **Time (min)** | **X MDB (%)** | **Y MDP1P (%)** |
|---|---|---|
| 40 | 50 | 25 |
| 80 | 60 | 27 |
| 120 | 30 | 20 |
| 160 | 36 | 20 |
| 200 | 26 | 17 |
| 290 | 28 | 18 |
| 320 | 29 | 22 |

| | | |
|---|---|---|
| Reaction conditions: MDB:AP=1:1; reaction temperature 150°C; flow 0,05 mL/min. | | |

This catalytic test performed in flow had the main goal to cover also this technical solution for the application of solid acid resins. The test demonstrated that the process of the invention could be advantageously conducted in a continuous way.

### Example 27

### Regeneration of PW87 catalyst with H₂O₂

2.1417 grams of MDB and 2.2994 grams of propionic anhydride were loaded inside a 25 mL two-necked round bottom flask and stirred to get a homogenous solution. After that 0.1004 grams of catalyst (AQUIVION PW87-S, corresponding to a mol ratio between active acid sites and MDB equal to 0.007), previously subjected to a micronization process to a particle size ranging from 40 µm to 100 µm as measured with DLS (Dynamic light scattering) or a granulometer was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20µL of octane was used as internal standard for the gas-chromatograph quantifications. The analysis of the products at the end of the reaction was carried out by a GC equipped with a HP-5 (Agilent) capillary column (the temperature ramp used consisted of 6 minutes at 35°C followed by heating with a speed of 10°C/min up to 280°C), and a flame ionization detector (FID). After that, the catalyst was regenerated as described in Example 9 using 5 mL of solution of H₂O₂ 30%w/w at 100°C for 20 min instead of mineral acid, recovered, washed and dried as previously reported and tested in the same condition for the acylation reaction. The results are reported in the following Table 15.

**Table 15., reaction conditions: molar ratio between active sites and MDB equal to 0.007; MDB:AP = 1:1, reaction temperature 80°C; reaction time 1h.**

| Cycle | X MDB (%) | X AP (%) | Y MDP1P (%) | Y ACP (%) | S MDP1P (%) | Y By-products |
|---|---|---|---|---|---|---|
| | 50 | 77 | 41 | 100 | 82 | 9 |
| 1 | 51 | 83 | 44 | 100 | 86 | 15 |

The catalytic activity was recovered with evident advantages in terms of process efficiency.

### Example 28

General procedure for the preparation of a supported catalyst with Aquivion (D72 or D79, or D82, or D98)

A mixture of Si (MeO)₄, distilled water, and 0.04M HCl with a weight ratio of 68:11:1 respectively was stirred for 45 min to give a clear solution. An Aquivion dispersion (D72 or D79, or D82, or D98) (which may contain for instance 25% w/w of resin in water or a mixture of water and alcohols), was diluted in water or water and alcohols. To the resin containing solution, was added a 0.4M NaOH aqueous solution, while stirring. The silicon containing solution prepared as described above was rapidly added to the stirred resin/NaOH solution to form a solid gel after few seconds after mixing. The solid was dried overnight in an oven at 95°C. After pulverization, the obtained powder was reacidified by stirring with a suitable amount of 3.5M HCl aqueous solution, and then washed with deionized water. This process was repeated 4 times. The catalyst was then treated with 25% w/w HNO₃ overnight at 75°C, washed with deionized water and dried at 100°C for 12h.

### Example 29

### Synthesis of catalysts containing 13wt% Aquivion supported into silica.

A mixture of 12.6706 g of Si(MeO)₄, 2.0497 g of distilled water, and 0.1863 g of 0.04M HCl was stirred for 45 min to give a clear solution. A mixture of 2.9712 g of Aquivion D72 (or D79, or D82, or D98) resin solution (which contains 25% w/w of resin), 8 g of distilled water, and 9.96 g of 1-propanol was prepared. To the resin containing solution, was added 10.3 mL of a 0.4M NaOH aqueous solution, while stirring. The silicon containing solution prepared as described above was rapidly added to the stirred resin/NaOH solution to form a solid gel after few seconds after mixing. The solid was dried overnight in an oven at 95°C. After grinding, the obtained powder having particle size ranging from 40 µm to 100 µm was reacidified by stirring with 50 mL of 3.5M HCl aqueous solution, and then washed with deionized water. This process was repeated 4 times. The catalyst was then treated with 25% w/w HNO₃ overnight at 75°C, washed with deionized water and dried at 100°C for 12h.

### Example 30

### Synthesis of catalysts containing 13wt% Nafion supported into silica.

A mixture of 7.6003 g of Si(MeO)₄, 1.2306 g of distilled water, and 0.1112 g of 0.04M HCl was stirred for 45 min to give a clear solution. A mixture of 2.2284 g of Nafion resin solution (which contains 20% w/w of resin), 3.34 g of distilled water, and 4.16 g of 1-propanol was prepared. To the resin containing solution, was added 5.52 mL of a 0.4M NaOH aqueous solution, while stirring. The silicon containing solution prepared as described above was rapidly added to the stirred resin/NaOH solution to form a solid gel after few seconds after mixing. The solid was dried overnight in an oven at 95°C. After grinding, the obtained powder having particle size ranging from 40 µm to 100 µm was reacidified by stirring with 50 mL of 3.5M HCl aqueous solution, and then washed with deionized water. This process was repeated 4 times. The catalyst was then treated with 25% w/w HNO₃ overnight at 75°C, washed with deionized water and dried at 100°C for 12h.

### Example 31

### Process of the invention with catalyst Aquivion D72 supported into silica.

2.1458 grams of MDB and 2.3081 grams of propionic anhydride were loaded inside a 25 mL two-necked round bottom flask and stirred to get a homogenous solution. After that 0.6726 grams of catalyst (Aquivion D72 13% w/w over silica, corresponding to a mol ratio between active acid sites and MDB equal to 0.007) produced with a particle size in range from 40 µm to 100 µm was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20µL of octane was used as internal standard for the gas-chromatograph quantifications. The analysis of the products at the end of the reaction was carried out by a GC equipped with a HP-5 (Agilent) capillary column (the temperature ramp used consisted of 6 minutes at 35°C followed by heating with a speed of 10°C/min up to 280°C), and a flame ionization detector (FID). The results were the following: MDB conversion of 59%, propionic anhydride conversion of 90%, 3,4-methylenedioxypropiophenone yield of 50% and selectivity of 85%, by-products yield of 9%.

### Example 32

### Acylation of the invention with catalyst Aquivion D79 supported into silica.

2.1354 grams of MDB and 2.2956 grams of propionic anhydride were loaded inside a 25 mL two-necked round bottom flask and stirred to get a homogenous solution. After that 0.7448 grams of catalyst (Aquivion D79 13% w/w over silica, corresponding to a mol ratio between active acid sites and MDB equal to 0.007), produced with a particle size in range from 40 µm to 100 µm, was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20µL of octane was used as internal standard for the gas-chromatograph quantifications. The analysis of the products at the end of the reaction was carried out by a GC equipped with a HP-5 (Agilent) capillary column (the temperature ramp used consisted of 6 minutes at 35°C followed by heating with a speed of 10°C/min up to 280°C), and a flame ionization detector (FID). The results were the following: MDB conversion of 50%, propionic anhydride conversion of 85%, 3,4-methylenedioxypropiophenone yield of 43% and selectivity of 86%, by-products yield of 7%.

### Example 33

### Acylation of the invention with catalyst Aquivion D83 supported into silica.

2.1402 grams of MDB and 2.3039 grams of propionic anhydride were loaded inside a 25 mL two-necked round bottom flask and stirred to get a homogenous solution. After that 0.7754 grams of catalyst (Aquivion D83 13% w/w over silica, corresponding to a mol ratio between active acid sites and MDB equal to 0.007), produced with a particle size in range from 40 µm to 100 µm, was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20µL of octane was used as internal standard for the gas-chromatograph quantifications. The analysis of the products at the end of the reaction was carried out by a GC equipped with a HP-5 (Agilent) capillary column (the temperature ramp used consisted of 6 minutes at 35°C followed by heating with a speed of 10°C/min up to 280°C), and a flame ionization detector (FID). The results were the following: MDB conversion of 51%, propionic anhydride conversion of 86%, 3,4-methylenedioxypropiophenone yield of 42% and selectivity of 83%, by-products yield of 7%.

### Example 34

### Process of the invention with catalyst Aquivion D98 supported into silica.

As a general procedure, 1.0673 grams of MDB and 1.1502 grams of propionic anhydride were loaded inside a 25 mL two-necked round bottom flask and stirred to get a homogenous solution. After that 0.4589 grams of catalyst (Aquivion D98 13% w/w over silica, corresponding to a mol ratio between active acid sites and MDB equal to 0.007), produced with a particle size in range from 40 µm to 100 µm, was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20µL of octane was used as internal standard for the gas-chromatograph quantifications. The analysis of the products at the end of the reaction was carried out by a GC equipped with a HP-5 (Agilent) capillary column (the temperature ramp used consisted of 6 minutes at 35°C followed by heating with a speed of 10°C/min up to 280°C), and a flame ionization detector (FID). The results were the following: MDB conversion of 49%, propionic anhydride conversion of 86%, 3,4-methylenedioxypropiophenone yield of 42% and selectivity of 85%, by-products

### Example 35

### Process of the invention with catalyst Aquivion D72 (5% w/w) supported into silica.

As a general procedure, 2.146 grams of MDB and 2.3044 grams of propionic anhydride were loaded inside a 25 mL two-necked round bottom flask and stirred to get a homogenous solution. After that 1.762 grams of catalyst obtained as described in Example 29 modifying the amount of resin loading (Aquivion D72 5% w/w over silica, corresponding to a mol ratio between active acid sites and MDB equal to 0.007), produced with a particle size in range from 40 µm to 100 µm, was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20µL of octane was used as internal standard for the gas-chromatograph quantifications. The analysis of the products at the end of the reaction was carried out by a GC equipped with a HP-5 (Agilent) capillary column (the temperature ramp used consisted of 6 minutes at 35°C followed by heating with a speed of 10°C/min up to 280°C), and a flame ionization detector (FID). The results were the following: MDB conversion of 39%, propionic anhydride conversion of 96%, 3,4-methylenedioxypropiophenone yield of 33% and selectivity of 86%, by-products yield of 3%.

### Example 36

### Process of the invention with catalyst Aquivion D72 (30% w/w) supported into silica.

As a general procedure, 2.152 grams of MDB and 2.3269 grams of propionic anhydride were loaded inside a 25 mL two-necked round bottom flask and stirred to get a homogenous solution. After that 0.2941 grams of catalyst obtained as described in Example 29 modifying the amount of resin loading (Aquivion D72 30% w/w over silica, corresponding to a mol ratio between active acid sites and MDB equal to 0.007), produced with a particle size in range from 40 µm to 100 µm, was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20µL of octane was used as internal standard for the gas-chromatograph quantifications. The analysis of the products at the end of the reaction was carried out by a GC equipped with a HP-5 (Agilent) capillary column (the temperature ramp used consisted of 6 minutes at 35°C followed by heating with a speed of 10°C/min up to 280°C), and a flame ionization detector (FID). The results were the following: MDB conversion of 47%, propionic anhydride conversion of 78%, 3,4-methylenedioxypropiophenone yield of 42% and selectivity of 88%, by-products yield of 8%.

### Example 37

### Process of the invention with catalyst Nafion (13% w/w) supported into silica.

As a general procedure, 1.4281 grams of MDB and 1.5415 grams of propionic anhydride were loaded inside a 25 mL two-necked round bottom flask and stirred to get a homogenous solution. After that 0.630 grams of catalyst obtained as described in Example 29 (Nafion 13% w/w over silica, corresponding to a mol ratio between active acid sites and MDB equal to 0.007), produced with a particle size in range from 40 µm to 100 µm, was inserted inside the liquid mixture and the flask was equipped with two condensers. The reaction mixture was then inserted in a pre-heated oil bath at 80°C and the reaction was performed under magnetic stirring (500 rpm) for 1 hour. After the reaction, the mixture was rapidly cooled in an ice bath and the reaction mixture was recovered in 50 mL of acetone HPLC grade. 0.8 mL of the mixture was diluted in 10 mL of acetone HPLC grade, 20µL of octane was used as internal standard for the gas-chromatograph quantifications. The analysis of the products at the end of the reaction was carried out by a GC equipped with a HP-5 (Agilent) capillary column (the temperature ramp used consisted of 6 minutes at 35°C followed by heating with a speed of 10°C/min up to 280°C), and a flame ionization detector (FID). The results were the following: MDB conversion of 49%, propionic anhydride conversion of 85%, 3,4-methylenedioxypropiophenone yield of 41 % and selectivity of 85%, by-products yield of 6%.

## Claims

1. A process for the preparation of 1-(1,3-benzodioxol-5-yl)-1-propanone comprising the step of reacting 3,4-methylendioxybenzene with propionic anhydride in the presence of a bulk or supported catalyst, being said catalyst selected from the group of sulphonated cross-linked polystyrene-divinylbenzene resins, sulphonated cross-linked polystyrene-divinylbenzene resins partially exchanged with iron, zinc or gallium, perfluorinated sulphonic resins, and perfluorinated sulphonic resins partially exchanged with iron, zinc or gallium, wherein said catalyst has an average particle size from 1 µm to 300 µm, preferably from 1 µm to 180 µm, more preferably from 1 µm to 100 µm as measured with DLS (Dynamic light scattering) or a granulometer.

2. The process according to claim 1, wherein the bulk or supported catalyst is a sulphonated cross-linked polystyrene-divinyl benzene resin used as bulk or supported on silica, said sulphonated cross-linked polystyrene-divinyl benzene resin being **characterized by** an acid loading expressed as mmol of group -SO₃H per g of material in the range from 2 to 6, preferably 2.5 to 5.5, more preferably of about 5.

3. The process according to claim 1, wherein the bulk or supported catalyst is a perfluorinated sulphonic acid resins used as bulk or supported on silica, said perfluorinated sulphonic acid resin being **characterized by** an acid loading expressed as mmol of group -SO₃H per g of material in the range from 0.2 to 2, preferably from 0.7 to 1.6, more preferably acid loading of about 1.2.

4. The process according to claim 1, wherein the bulk or supported catalyst is a sulphonated cross-linked polystyrene-divinyl benzene resin or a perfluorinated sulphonic acid resin partially exchanged with iron or zinc or gallium, wherein the partial exchange is a percentage of hydrogen atom from 5 to 80% exchanged with a metal ion selected from Fe, Zn and Ga, preferably the exchange percentage is from 10 to 50%, more preferably it is about 30%.

5. The process according to anyone of claims 1-4, wherein the catalyst is supported over suitable inorganic oxides, preferably selected from zirconia, alumina, titania and silica, more preferably silica.

6. The process according to anyone of claims 1, 3-5 wherein the step of reacting 3,4-methylendioxybenzene with propionic anhydride is carried out in the range of temperature from 50 to 200°C, preferably 60 to 150°C, more preferably about 80 °C.

7. The process according to anyone of claims 1, 2, 4, 5, wherein the step of reacting 3,4-methylendioxybenzene with propionic anhydride is carried out in the range of temperature from 50 to 150°C, preferably 60 to 130°C, more preferably about 80°C or about 120 °C.

8. The process according to anyone of claims 1, 3-6, wherein the amount of catalyst expressed as ratio between mol of H+ or of active sites per mol of limiting reagent is from 0.0001 to 1, preferably from 0.001 to 0.04, more preferably about 0.007.

9. The process according to anyone of claims 1, 2, 4, 5, 7, wherein the amount of catalyst expressed as ratio between mol of H+ or of active sites per mol of limiting reagent is from 0.002 to 1, more preferably from 0.01 to 0.08, still more preferably about 0.03.

10. The process according to anyone of claims 1-9, wherein the step of reacting 3,4-methylendioxybenzene with propionic anhydride is carried out with a molar ratio between 3,4-methylendioxybenzene and propionic anhydride is in the range from 2 to 1 to 1 to 2, preferably in 1:1 stoichiometric ratio.

11. The process according to anyone of claims 1-10, wherein the catalyst is recovered, regenerated and recycled.

12. The process according to claim 11, wherein the catalyst is recovered by filtration from the reaction medium and suspension in a diluted acid aqueous solution of nitric acid, preferably at a concentration in the range from 2%wt to 60%wt, more preferably from 10 to 40%, still more preferable of 20%wt.

13. The process according to claim 11, wherein the catalyst is recovered by filtration from the reaction medium and suspension in a diluted acid aqueous solution of hydrochloric acid, preferably at a concentration in the range from 2% wt to 30% wt, more preferably 20%wt.

14. The process according to claim 11, wherein the recovery, the regeneration and the recycle of the catalyst is carried out with the use of H₂O₂.

15. The process according to anyone of claims 1-14 wherein the process is a continuous process.

## Patentansprüche

1. Verfahren zur Herstellung von 1-(1,3-Benzodioxol-5-yl)-1-propanon, umfassend den Schritt des Reagierens von 3,4-Methylendioxybenzol mit Propionsäureanhydrid in Gegenwart eines Schütt- oder geträgerten Katalysators, wobei der Katalysator aus der Gruppe der sulfonierten vernetzten Polystyrol-Divinylbenzolharze, sulfonierten vernetzten Polystyrol-Divinylbenzolharze, die teilweise mit Eisen, Zink oder Gallium ausgetauscht sind, perfluorierten Sulfonsäureharze und perfluorierten Sulfonsäureharze, die teilweise mit Eisen, Zink oder Gallium ausgetauscht sind, ausgewählt ist, wobei der Katalysator eine durchschnittliche Teilchengröße von 1 µm bis 300 µm, vorzugsweise von 1 µm bis 180 µm, bevorzugter von 1 µm bis 100 µm, aufweist, gemessen mit DLS (dynamischer Lichtstreuung) oder einem Granulometer.

2. Verfahren nach Anspruch 1, wobei der Schütt- oder geträgerte Katalysator ein sulfoniertes vernetztes Polystyrol-Divinylbenzolharz ist, das in Schüttung oder auf Siliziumoxid geträgert verwendet wird, wobei das sulfonierte vernetzte Polystyrol-Divinylbenzolharz durch eine Säurebeladung, die als mmol der Gruppe -SO₃H pro g Material ausgedrückt wird, im Bereich von 2 bis 6, vorzugsweise 2,5 bis 5,5, bevorzugter etwa 5, gekennzeichnet ist.

3. Verfahren nach Anspruch 1, wobei der Schütt- oder geträgerte Katalysator perfluorierte Sulfonsäureharz ist, das in Schüttung oder auf Siliziumoxid geträgert verwendet wird, wobei das perfluorierte Sulfonsäureharz durch eine Säurebeladung, die als mmol der Gruppe -SO₃H pro g Material ausgedrückt wird, im Bereich von 0,2 bis 2, vorzugsweise 0,7 bis 1,6, bevorzugter eine Säurebeladung von etwa 1,2, gekennzeichnet ist.

4. Verfahren nach Anspruch 1, wobei der Schütt- oder geträgerte Katalysator ein sulfoniertes vernetztes Polystyrol-Divinylbenzolharz oder ein perfluoriertes Sulfonsäureharz ist, das teilweise mit Eisen oder Zink oder Gallium ausgetauscht ist, wobei der teilweise Austausch ein Prozentsatz an Wasserstoffatomen von 5 bis 80 % ist, die mit einem Metallion ausgetauscht sind, das aus Fe, Zn und Ga ausgewählt ist, wobei der Austauschprozentsatz vorzugsweise 10 bis 50 %, bevorzugter etwa 30 %, beträgt.

5. Verfahren nach einem der Ansprüche 1-4, wobei der Katalysator auf geeigneten anorganischen Oxiden, vorzugsweise ausgewählt aus Zirkonoxid, Aluminiumoxid, Titanoxid und Siliziumoxid, bevorzugter Siliziumoxid, geträgert ist.

6. Verfahren nach einem der Ansprüche 1, 3-5, wobei der Schritt des Reagierens von 3,4-Methylendioxybenzol mit Propionsäureanhydrid in dem Temperaturbereich von 50 bis 200 °C, vorzugsweise von 60 bis 150 °C, bevorzugter etwa 80 °C, durchgeführt wird.

7. Verfahren nach einem der Ansprüche 1, 2, 4, 5, wobei der Schritt des Reagierens von 3,4-Methylendioxybenzol mit Propionsäureanhydrid in dem Temperaturbereich von 50 bis 150 °C, vorzugsweise von 60 bis 130 °C, bevorzugter etwa 80 °C oder etwa 120 °C, durchgeführt wird.

8. Verfahren nach einem der Ansprüche 1, 3-6, wobei die Menge an Katalysator, die als Verhältnis zwischen Mol H+ oder aktiven Stellen pro Mol beschränkendes Reagenz ausgedrückt wird, von 0,0001 bis 1, vorzugsweise von 0,001 bis 0,04, bevorzugter etwa 0,007, beträgt.

9. Verfahren nach einem der Ansprüche 1, 2, 4, 5, 7, wobei die Menge an Katalysator, die als Verhältnis zwischen Mol H+ oder aktiven Stellen pro Mol beschränkendes Reagenz ausgedrückt wird, von 0,002 bis 1, bevorzugter von 0,01 bis 0,08, noch bevorzugter etwa 0,03, beträgt.

10. Verfahren nach einem der Ansprüche 1-9, wobei der Schritt des Reagierens von 3,4-Methylendioxybenzol mit Propionsäureanhydrid bei einem Molverhältnis von 3,4-Methylendioxybenzol zu Propionsäureanhydrid im Bereich von 2 zu 1 bis 1 zu 2, vorzugsweise in einem stöchiometrischen Verhältnis von 1 : 1, durchgeführt wird.

11. Verfahren nach einem der Ansprüche 1-10, wobei der Katalysator rückgewonnen, regeneriert und wiederverwendet wird.

12. Verfahren nach Anspruch 11, wobei der Katalysator durch Filtration aus dem Reaktionsmedium und Suspension in einer verdünnten sauren wässrigen Lösung von Salpetersäure, vorzugsweise in einer Konzentration im Bereich von 2 Gew.-% bis 60 Gew.-%, bevorzugter von 10 bis 40 Gew.-%, noch bevorzugter von 20 Gew.-%, rückgewonnen wird.

13. Verfahren nach Anspruch 11, wobei der Katalysator durch Filtration aus dem Reaktionsmedium und Suspension in einer verdünnten sauren wässrigen Lösung von Salzsäure, vorzugsweise in einer Konzentration im Bereich von 2 Gew.-% bis 30 Gew.-%, bevorzugter 20 Gew.-%, rückgewonnen wird.

14. Verfahren nach Anspruch 11, wobei die Rückgewinnung, die Regeneration und die Wiederverwendung des Katalysators unter Verwendung von H₂O₂ durchgeführt wird.

15. Verfahren nach einem der Ansprüche 1-14, wobei das Verfahren ein kontinuierliches Verfahren ist.

## Revendications

1. Procédé de préparation de 1-(1,3-benzodioxol-5-yl)-1-propanone comprenant l'étape de réaction du 3,4-méthylènedioxybenzène avec l'anhydride propionique en présence d'un catalyseur en vrac ou supporté, ledit catalyseur étant choisi du groupe des résines polystyrène-divinylbenzène réticulées sulfonées, des résines polystyrène-divinylbenzène réticulées sulfonées partiellement échangées avec le fer, le zinc ou le gallium, des résines sulfoniques perfluorées, et des résines sulfoniques perfluorées partiellement échangées avec le fer, le zinc ou le gallium, dans lequel ledit catalyseur a une granulométrie moyenne de 1 µm à 300 µm, de préférence de 1 µm à 180 µm, plus préférentiellement de 1 µm à 100 µm telle que mesurée par DLS (diffusion dynamique de la lumière, en Anglais Dynamic light scattering) ou un granulomètre.

2. Procédé selon la revendication 1, dans lequel le catalyseur en vrac ou supporté est une résine de polystyrène-divinylbenzène réticulée sulfonée utilisée en vrac ou supportée sur de la silice, ladite résine de polystyrène-divinylbenzène réticulée sulfonée étant **caractérisée par** une charge acide exprimée en mmol du groupe-SO₃H chaque g de matière comprise entre 2 et 6, de préférence entre 2,5 et 5,5, plus préférablement d'environ 5.

3. Procédé selon la revendication 1, dans lequel le catalyseur en vrac ou supporté est des résines d'acide sulfonique perfluoré utilisée en vrac ou supportée sur de la silice, ladite résine d'acide sulfonique perfluoré étant **caractérisée par** une charge acide exprimée en mmol du groupe -SO₃H chaque g de matière comprise entre 0,2 et 2, de préférence entre 0,7 et 1,6, plus préférablement une charge acide d'environ 1,2.

4. Procédé selon la revendication 1, dans lequel le catalyseur en vrac ou supporté est une résine de polystyrène-divinylbenzène réticulée sulfonée ou une résine d'acide sulfonique perfluoré partiellement échangée avec du fer ou du zinc ou du gallium, dans lequel l'échange partiel est un pourcentage d'atome d'hydrogène de 5 à 80 % échangé avec un ion métallique choisi parmi Fe, Zn et Ga, de préférence le pourcentage d'échange est de 10 à 50 %, plus préférablement il est environ le 30 %.

5. Procédé selon l'une quelconque des revendications 1 à 4, dans lequel le catalyseur est supporté sur des oxydes inorganiques appropriés, de préférence choisis parmi la zircone, l'alumine, l'oxyde de titane et la silice, plus préférablement la silice.

6. Procédé selon l'une quelconque des revendications 1, 3 à 5, dans lequel l'étape de réaction du 3,4-méthylendioxybenzène avec l'anhydride propionique est effectuée dans la plage de température de 50 à 200 °C, de préférence de 60 à 150 °C, plus préférablement d'environ 80 °C.

7. Procédé selon l'une quelconque des revendications 1, 2, 4, 5, dans lequel l'étape de réaction de 3,4-méthylendioxybenzène avec l'anhydride propionique est effectuée dans la plage de température de 50 à 150 °C, de préférence de 60 à 130 °C, plus préférablement d'environ 80 °C ou d'environ 120 °C.

8. Procédé selon l'une quelconque des revendications 1, 3 à 6, dans lequel la quantité de catalyseur exprimée en rapport entre les moles de H+ ou de sites actifs par mole de réactif limitant est de 0,0001 à 1, de préférence de 0,001 à 0,04, plus préférablement d'environ 0,007.

9. Procédé selon l'une quelconque des revendications 1, 2, 4, 5, 7, dans lequel la quantité de catalyseur exprimée en rapport entre les moles de H+ ou de sites actifs par mole de réactif limitant est de 0,002 à 1, plus préférablement de 0,01 à 0,08, encore plus préférablement d'environ 0,03.

10. Procédé selon l'une quelconque des revendications 1 à 9, dans lequel l'étape de réaction du 3,4-méthylènedioxybenzène avec l'anhydride propionique est effectuée avec un rapport molaire entre le 3,4-méthylènedioxybenzène et l'anhydride propionique qui est dans la plage de 2 à 1 à 1 à 2, de préférence dans un rapport stœchiométrique de 1:1.

11. Procédé selon l'une quelconque des revendications 1 à 10, dans lequel le catalyseur est récupéré, régénéré et recyclé.

12. Procédé selon la revendication 11, dans lequel le catalyseur est récupéré par filtration à partir du milieu réactionnel et de la suspension dans une solution aqueuse acide diluée d'acide nitrique, de préférence à une concentration comprise entre 2 % en poids et 60 % en poids, plus préférablement entre 10 et 40 %, encore plus préférablement de 20 % en poids.

13. Procédé selon la revendication 11, dans lequel le catalyseur est récupéré par filtration à partir du milieu réactionnel et de la suspension dans une solution aqueuse d'acide diluée d'acide chlorhydrique, de préférence à une concentration comprise entre 2 % en poids et 30 % en poids, plus préférablement 20 % en poids.

14. Procédé selon la revendication 11, dans lequel la récupération, la régénération et le recyclage du catalyseur sont effectués à l'aide de H₂O₂.

15. Procédé selon l'une quelconque des revendications 1 à 14, dans lequel le procédé est un procédé continu.
